# EUROPEAN PATENT APPLICATION

(11) **EP 4 450 613 A1**
(43) Date of publication of application: **23.10.2024**
(21) Application number: 23746844.2
(22) Date of filing: 20.01.2023
(51) Int. Cl.: C12N 5/076, A61K 31/437, A61K 31/522, A61P 15/08

(54) **MAMMALIAN SPERM PREPARATION METHOD, ARTIFICIAL INSEMINATION METHOD, AND IN-VITRO FERTILIZATION METHOD**

(30) Priority: 26.01.2022 JP 2022009945
(71) Applicant: Hiroshima University, Higashihiroshima-shi Hiroshima 739-8511 (JP)
(72) Inventor: SHIMADA Masayuki, Higashihiroshima-shi, Hiroshima 739-8511 (JP); UMEHARA Takashi, Higashihiroshima-shi, Hiroshima 739-8511 (JP)
(74) Representative: Epping - Hermann - Fischer
(86) International application number: PCT/JP2023/001637
(87) International publication number: WO 2023/145631

(57) **Abstract**

In a method of preparing mammalian sperm, mammalian sperm are cultured in a medium at a pH of 7.7 to 7.9 containing a TLR7 ligand, to induce acrosome reaction of Y-chromosome-bearing sperm, to obtain a sperm population rich in X-chromosome-bearing sperm retaining the acrosome. By subjecting the sperm population to artificial insemination or in vitro fertilization, gender preselection for obtaining male offspring is possible.

## Description

### Technical Field

The present disclosure relates to a method of preparing mammalian sperm, an artificial insemination method, and an in-vitro fertilization method.

### Background Art

Techniques for gender preselection in domestic animals greatly contribute to efficient production of the domestic animals. For example, in the case of cows, planned preselection for females enables acquisition of succeeding cows. For milk production, cows need to be kept pregnant. After the acquisition of the succeeding cows, embryos of Japanese Black Cattle, whose calves are high-priced, may be transferred to the cows to produce Japanese Black Cattle. By this, dairy farmers can achieve better management.

In swine production, castrated males show rapid growth, and their fattening period for reaching the market weight is shorter than that of females. Therefore, in the production of pork pigs, production of males not only provides better management for pig farmers, but also contributes to increasing the world's food production by increasing the pork production per feed. In the pig production, castration has long been performed without anesthesia. However, recently, castration without anesthesia was prohibited in Europe and Canada from an animal welfare perspective, and the production cost has inevitably increased in these countries. Since meat of male pigs without castration has low fat content, and emits the unfavorable odor called "male odor", its value is very low. Therefore, selective production of females is expected to be effective also for suppressing the increase in the production cost in the countries where castration without anesthesia is prohibited.

Thus, in the livestock industry, techniques for gender preselection are highly demanded, and examples of known techniques for the gender preselection include a method according to Patent Literature 1.

Patent Literature 1 focuses on the fact that Toll-like receptors 7 and 8 (TLR7/8) are expressed only in X-chromosome-bearing sperm. By culturing sperm in a medium supplemented with an agent that activates TLR7/8, X-chromosome-bearing sperm, which have decreased motility, are separated from Y-chromosome-bearing sperm, which have good motility.

### Citation List

### Patent Literature

Patent Literature 1: Unexamined Japanese Patent Application Publication No. 2019-10094

### Summary of Invention

### Technical Problem

Patent Literature 1 enables efficient separation between X-chromosome-bearing sperm and Y-chromosome-bearing sperm. However, in cases where X-chromosome-bearing sperm are to be used for artificial insemination or in vitro fertilization, the motility of the X-chromosome-bearing sperm, which has once been decreased by the agent, needs to be restored after the collection of the X-chromosome-bearing sperm. Since the restoration requires a step of repeated centrifugal washing, additional culture, or the like, it is too complicated to be carried out at the sites of animal husbandry.

The present disclosure was carried out in view of the above circumstances, and an objective of the present disclosure is to provide a simple method of preparing mammalian sperm by which a sperm population rich in X-chromosome-bearing sperm retaining the acrosome can be obtained, an artificial insemination method, and an in-vitro fertilization method.

### Solution to Problem

A method of preparing mammalian sperm according to a first aspect of the present disclosure is characterized in that mammalian sperm are cultured in a medium at a pH of 7.7 to 7.9 containing a TLR7 ligand, to induce the acrosome reaction of Y-chromosome-bearing sperm, to obtain a sperm population rich in X-chromosome-bearing sperm retaining the acrosome.

The medium preferably contains albumin.

The medium preferably contains neither pyruvic acid nor lactic acid.

The culture is preferably carried out for a period longer than 30 minutes and shorter than 90 minutes.

The TLR7 ligand is preferably one or more selected from the group consisting of resiquimod, imiquimod, gardiquimod, and loxoribine.

An artificial insemination method according to a second aspect of the present disclosure is characterized in that artificial insemination of a non-human mammal is carried out using non-human-mammalian sperm prepared by the method according to the first aspect of the present disclosure.

An in-vitro fertilization method according to a third aspect of the present disclosure is characterized in that in vitro fertilization of a non-human mammal is carried out using non-human-mammalian sperm prepared by the method according to the first aspect of the present disclosure.

### Advantageous Effects of Invention

The present disclosure can provide a simple method of preparing mammalian sperm by which a sperm population rich in X-chromosome-bearing sperm retaining the acrosome can be obtained, an artificial insemination method, and an in-vitro fertilization method.

### Brief Description of Drawings

FIG. 1 is a graph illustrating the acrosome retention rate in Experiment 1;
FIG. 2 is a graph illustrating the acrosome retention rate in Experiment 2;
FIG. 3 is a graph illustrating the acrosome retention rate in Experiment 3;
FIG. 4 is a graph illustrating the acrosome retention rate in Experiment 4;
FIG. 5 is a graph illustrating the ratio of X-sperm in Experiment 5;
FIG. 6 is a graph illustrating the acrosome retention rate in Experiment 7;
FIG. 7 is a graph illustrating the acrosome retention rate in Experiment 9;
FIG. 8 is a graph illustrating the ratios of X-sperm and Y-sperm in Experiment 9; and
FIG. 9 is a graph illustrating the ratios of XX-embryos and XY-embryos in Experiment 10.

### Description of Embodiments

In the method of preparing mammalian sperm according to the present embodiment, mammalian sperm are cultured in a medium at a pH of 7.7 to 7.9 containing a TLR7 ligand, to induce the acrosome reaction of Y chromosome-bearing sperm, to obtain a sperm population rich in X-chromosome-bearing sperm retaining the acrosome. In the present description, X-chromosome-bearing sperm are referred to as "X-sperm", and Y-chromosome-bearing sperm are referred to as "Y-sperm".

After being ejaculated into a female's accessory reproductive organ, a sperm swims in a manner dependent on the ambient environment, and then reaches the oviduct, where fertilization occurs. In the oviduct, the sperm meets an egg, and this causes breakage of the acrosome, which is present at the sperm head. As a result, enzyme secretion occurs. The acrosome reaction is essential for the sperm to enter the egg, to complete fertilization. The acrosome reaction may occur spontaneously even outside the oviduct, and sperm that have caused the acrosome reaction in the early stage are phagocytized in the uterus, and hence cannot reach the oviduct. Thus, for the completion of fertilization, it is essential for a sperm to retain the acrosome until the sperm reaches the oviduct, and to cause the acrosome reaction at the appropriate time.

In cases where an X-sperm enters an egg to complete fertilization, a female embryo is obtained to produce a female offspring. In cases where a Y-sperm enters an egg to complete fertilization, a male embryo is obtained to produce a male offspring.

In the present embodiment, culturing of sperm in a medium at a pH of 7.7 to 7.9 containing a TLR7 ligand leads to increased sperm metabolism of Y-sperm, to induce the acrosome reaction. The Y-sperm thus loses the acrosome, and hence loses the fertility. On the other hand, the sperm metabolism of X-sperm is suppressed in the above medium, and the acrosome is therefore retained, so that the fertility of X-sperm is secured. As a result, in the cultured sperm population, most sperm retaining the acrosome are X-sperm. Thus, a sperm population rich in X-sperm retaining the acrosome is obtained.

The medium containing a TLR7 ligand may be prepared by adding the TLR7 ligand to a basal medium. The basal medium may be a medium that can be normally used for the culture of mammalian sperm. Preferred examples of the basal medium include embryo culture media such as human tubal fluid (HTF) medium (composition: NaCl, KCl, KH₂PO₄, MgSO₄·7H₂O, CaCl₂·2H₂O, NaHCO₃, glucose, Na-pyruvate, Na-lactate, gentamicin sulfate salt, and phenol red).

The pH of the medium is 7.7 to 7.9. In cases where the pH is not within this range, the significant difference in the acrosome retention rate between X-sperm and Y-sperm is small as shown in the Examples below. The pH can be adjusted by adding a pH adjusting agent such as NaOH to the medium.

The medium preferably contains albumin. In cases where sperm are cultured in a medium not containing albumin, the sperm metabolism is suppressed in both X-sperm and Y-sperm as a result. Therefore, in cases where sperm are cultured in a medium that does not contain albumin, but contains the TLR7 ligand, the sperm metabolism of Y-sperm is also suppressed to cause reduction of the loss of the acrosome, so that the sperm population rich in X-sperm retaining the acrosome cannot be easily obtained. Examples of the albumin include bovine serum albumin (BSA). The albumin content in the medium is preferably about 2 to 8 g/L, more preferably about 5 g/L.

The medium preferably contains neither pyruvic acid nor lactic acid. In cases where the basal medium contains neither pyruvic acid nor lactic acid, the acrosome retention rate of X-sperm can be further increased.

Examples of the TLR7 ligand to be added include resiquimod, imiquimod, gardiquimod, and loxoribine. These may be used individually, or two or more of these may be used.

The amount of the TLR7 ligand added to the basal medium may be appropriately set in accordance with the type of TLR7 ligand used and the animal species of the sperm used. For example, the amount is preferably 3 nM to 3 µM.

The culture period is preferably a period sufficient for the activation of Y-sperm. For example, the culture period is preferably longer than 30 minutes. In cases where the culture period is too long, acrosome loss proceeds also in X-sperm.
Therefore, the culture period is preferably shorter than 90 minutes. The culture period is more preferably 60 minutes.

Since the present embodiment is carried out by culturing sperm in a medium containing a TLR7 ligand as described above, a large number of sperm can be treated.

As described above, the cultured sperm population is rich in X-sperm having the X chromosome and retaining the acrosome. Thus, in cases where artificial insemination or in vitro fertilization of a mammal is carried out using this sperm population, fertilization of X-sperm with eggs occurs to produce fertilized eggs having the XX chromosomes. Therefore, gender preselection for obtaining female offspring is possible. The artificial insemination or the in vitro fertilization may be carried out by a normal method.

In the cultured sperm population, X-sperm and Y-sperm may or may not be separated from each other. Since most of the Y sperm contained in the cultured sperm population lack the acrosome, and hence are in an infertile state, female offspring can be produced at a high ratio by subjecting the sperm population to artificial insemination or in vitro fertilization without separating X-sperm and Y-sperm from each other.

Thus, since the method of preparing mammalian sperm according to the present embodiment enables simple production of a sperm population rich in X-sperm without requiring laborious steps such as additional culture, the method can be easily carried out even at the sites of animal husbandry.

### Examples

### (Experiment 1) Test of Effect of Albumin

Semen was collected from a pig, and a dilution of the semen was centrifuged (300×G, 3 minutes) on the next day. BSA was added to HTF medium, to prepare modified HTF medium. The composition of the prepared modified HTF medium is illustrated in Table 1.

**[Table 1]**

| | mg/ 50 mL |
|---|---|
| NaCl | 271.905 |
| KCL | 17.482 |
| CaCl₂·2H₂O | 37.787 |
| KH₂PO₄ | 2.722 |
| MgSO₄·7H₂O | 2.465 |
| Phenol red | 0.1 |
| NaHCO₃ | 105 |
| D-glucose | 25.05 |
| Na-lactate (µL) | 174.5 |
| Pyruvic acid | 1.87 |
| Streptomycin | 2.5 |
| Penicillin G | 3.13 |
| 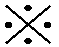 BSA | 250 |
| HEPEs (25 mM) | 297.9 |

To each of HTF medium and modified HTF medium, the pig semen was added, and culture was performed in a thermostat (37°C, 60 minutes). Thereafter, sperm were stained with peanut agglutinin fluorescein isothiocyanate (PNA-FITC), which is a marker for acrosome retention, and podium iodide (PI), which is a marker for cell survival, and washed by centrifugation. The sperm were then subjected to flow cytometry, and the ratio of PNA-/PI- sperm (sperm that are surviving, and retaining the acrosome) was calculated to determine the acrosome retention rate.

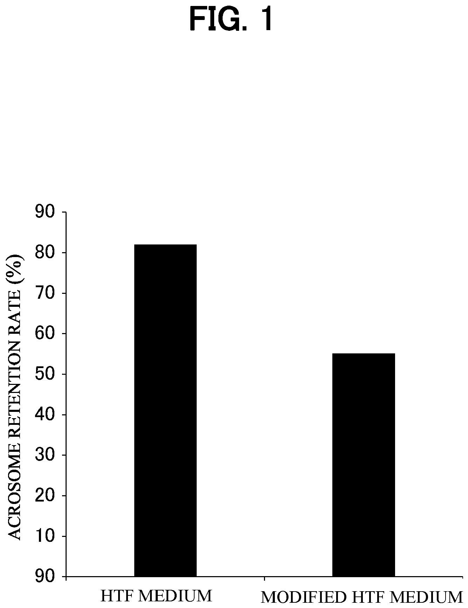

The results are illustrated in FIG. 1. The ratio of sperm retaining the acrosome was significantly lower in modified HTF medium than in HTF medium. It could be confirmed that culturing of pig sperm in a medium supplemented with albumin results in a decrease in the acrosome retention rate.

### (Experiment 2) Test of Effects of pH of Medium and R848 on Retention of Pig Sperm Acrosome

NaOH was added to modified HTF medium to adjust the pH to various values. In addition, resiquimod R-848 (hereinafter referred to as R848) (3 nM) as a TLR7 ligand was added thereto.

By the same method as in Experiment 1, pig sperm were cultured using each type of modified HTF medium, and the acrosome retention rate was determined. In addition, for comparison, the same experiment was carried out using the medium not supplemented with R848 (non-addition group).

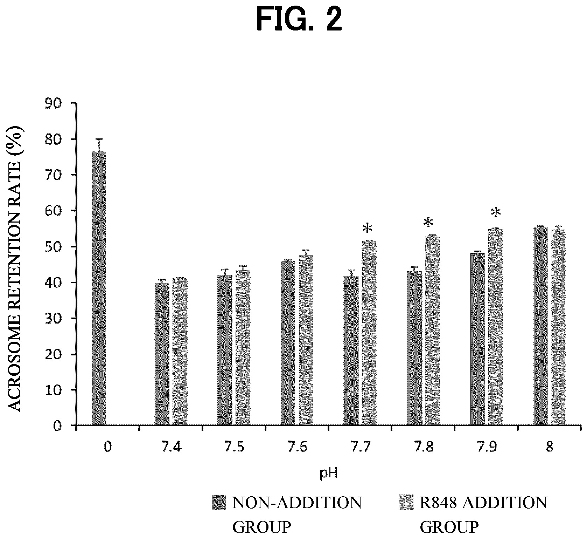

The results are illustrated in FIG. 2. At the pHs within the range of 7.7 to 7.9, the ratio of sperm retaining the acrosome was significantly higher in the R848 addition group than in the non-addition group. Minute 0 represents a case where PNA-FITC and PI were added to the diluted semen, and then the resulting mixture was left to stand in an environment at 15°C for 30 minutes.

### (Experiment 3) Test of Effect of Culture Period on Retention of Pig Sperm Acrosome

Pig sperm were cultured by the same method as in Experiment 1 except that modified HTF medium whose pH was adjusted to 7.8 and to which R848 was added (3 nM) was used, and that the culture period was changed. The acrosome retention rate was then determined (R848 addition group). In addition, for comparison, the same experiment was carried out using the medium not supplemented with R848 (non-addition group). The culture period was 0 minutes, 30 minutes, 60 minutes, or 90 minutes.

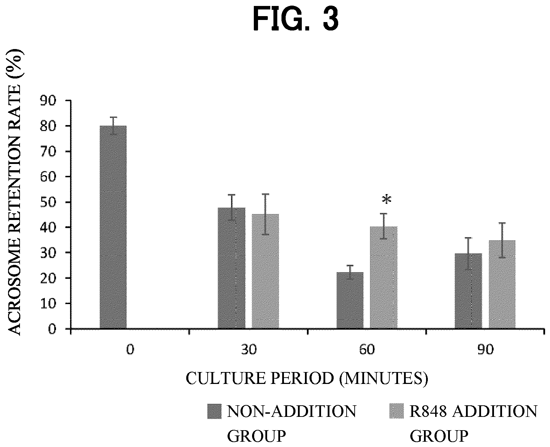

The results are illustrated in FIG. 3. The effect that significantly increases the ratio of sperm having a normal acrosome in the R848 addition group relative to that in the non-addition group was not found by Minute 30, but was significantly found at Minute 60. However, 90 minutes of the culture caused the acrosome reaction also in the R848 addition group, leading to the same retention rate as in the non-addition group. Thus, it can be seen that the culture period is preferably longer than 30 minutes and shorter than 90 minutes, and that the culture period is more preferably 60 minutes.

### (Experiment 4) Test of Effect of Energy Substrate in Medium on Retention of Pig Sperm Acrosome.

Modified HTF medium contains not only glucose, but also energy substrates such as pyruvic acid and lactic acid. In view of this, the effects of these energy substrates were tested.

Pig sperm were cultured by the same method as in Experiment 1 except that the pH was adjusted to 7.8, that R848 was added (3 nM), and that modified HTF media containing different combinations of energy substrates were used. The acrosome retention rate was then determined (R848 addition group). In addition, for comparison, the same experiment was carried out using modified HTF media not supplemented with R848 (non-addition group).

The modified HTF media used were as follows.
- Free medium: Medium containing the components listed in Table 1 except for glucose (D-glucose), pyruvic acid (Pyruvic acid), and lactic acid (Na-lactate)
- Glu medium: Medium containing the components listed in Table 1 except for pyruvic acid (Pyruvic acid) and lactic acid (Na-lactate)
- Pyr medium: Medium containing the components listed in Table 1 except for glucose (D-glucose) and lactic acid (Na-lactate)
- Lac medium: Medium containing the components listed in Table 1 except for glucose (D-glucose) and pyruvic acid (Pyruvic acid)
- All medium: Medium containing all components listed in Table 1

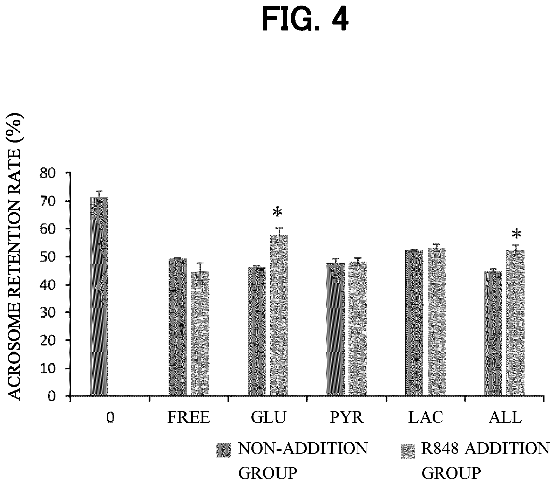

The results are illustrated in FIG. 4. The acrosome retention effect by R848 was found in the Glu medium. However, the acrosome retention effect by R848 was not found in the Free medium, the Pyr medium, and the Lac medium. It can thus be seen that the acrosome retention effect increases in a medium containing R848, but containing neither pyruvic acid nor lactic acid.

### (Experiment 5) Separation of X-Sperm

Sperm lacking the acrosome show a circular turning movement pattern, and are less likely to swim up. In view of this, separation of X-sperm was attempted by the swim-up.

Pig sperm were cultured (1 hour, 37°C) by the same method as in Experiment 1 except that modified HTF medium (Glu medium, pH 7.8) supplemented with R848 was used. After the culture, the reacted sperm were suspended in 2% polyvinylpyrrolidone (PVP) (1 mL), which is viscous, and the resulting suspension was placed under 2 mL of modified HTF medium to form a lower layer, followed by collecting sperm that swam up into the modified HTF medium 30 minutes thereafter.

The collected sperm were subjected to PNA staining, and the amount of fluorescence was quantified by flow cytometry. The sperm that remained in the lower layer were also similarly subjected to quantification of the amount of fluorescence.

Based on the results, it was confirmed that most sperm that swam up into the upper layer retained the acrosome similarly to the sperm before the culture, and that the sperm that remained in the lower layer had a damaged acrosome.

The ratio of X-sperm among the sperm collected from the upper layer was measured by real-time PCR (R848 addition group). For comparison, the same experiment was carried out using modified HTF medium not supplemented with R848, to measure the ratio of X-sperm (non-addition group).

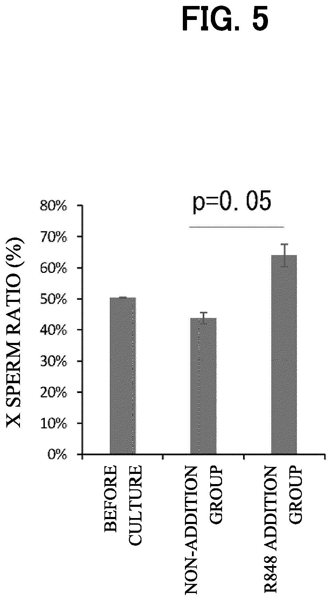

The results are illustrated in FIG. 5. The X-sperm rate among the sperm that swam up was significantly higher in the R848 addition group than in the non-addition group. Thus, most of the sperm that was retaining the acrosome was found to be X-sperm.

### (Experiment 6) Pig Artificial Insemination

Pig semen diluted to 5 billion/100 mL with Hiro Swine B solution was centrifuged (500 G, 5 minutes, room temperature), and then the supernatant was discarded. The pig sperm were suspended in the same amount of modified HTF medium (pH 7.8, supplemented with R848 (3 nM)), and then cultured in a thermostat (60 minutes, 37°C). Thereafter, centrifugation (500 G, 5 minutes, room temperature) was carried out, and the supernatant was discarded. Subsequently, the sperm were suspended in a diluent for artificial insemination, and then subjected to artificial insemination.

The results are illustrated in Table 2. In the non-addition group, 25 individuals were male, and 25 individuals were female out of 50 individuals, indicating that the male-female ratio was 50:50. However, in the case where the artificial insemination was carried out with the semen treated with the medium supplemented with R848, 13 individuals were male, and 23 individuals were female out of 36 individuals, indicating that females were obtained at a ratio of 64%.

**[Table 2]**

| | Male | Female | Female ratio |
|---|---|---|---|
| Non-addition group | 25 | 25 | 50% |
| R848 addition group | 13 | 23 | 64% |

### (Experiment 7) Test of Mouse Sperm

Sperm were collected from the cauda epididymis of 12-week-old mature male mice. The mouse sperm were cultured in modified HTF medium (Glu medium, pH 7.8, supplemented with R848 (3 nM)) (60 minutes, 37°C).

The cultured mouse sperm were fixed, and subjected to PNA-FITC staining, followed by measuring the acrosome retention rate by flow cytometry (R848 addition group). In addition, for comparison, an experiment was carried out in the same manner as described above except that a medium not supplemented with R848 was used, to measure the acrosome retention rate (non-addition group).

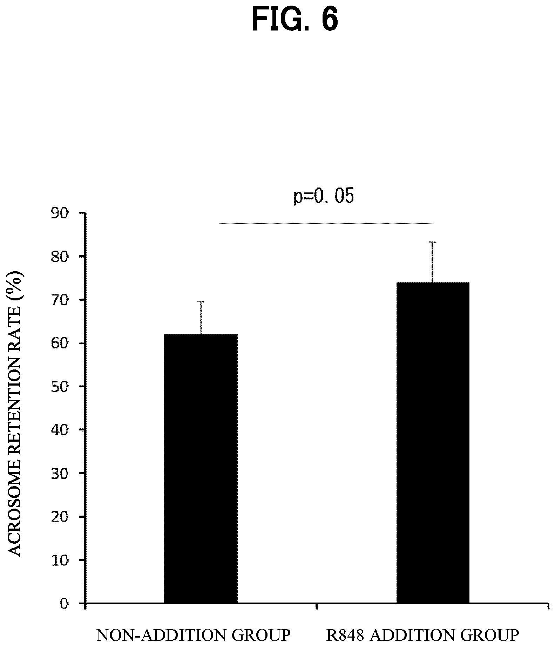

The results are illustrated in FIG. 6. Also in the case of the mouse sperm, the acrosome retention rate was significantly higher in the R848 addition group than in the non-addition group.

### (Experiment 8) In Vitro Fertilization and Artificial Insemination in Mice

In vitro fertilization was performed using sperm cultured in the same manner as in Experiment 7. Five days later, sperm that reached blastocyst embryos were collected, and the ratio between X-sperm and Y-sperm was calculated by real-time PCR.

The results are illustrated in Table 3. In the non-addition group, 8 individuals were male, and 8 individuals were female out of 16 individuals, indicating that the male-female ratio was 50:50. However, in the R848 addition group, 5 individuals were male, and 17 individuals were female out of 22 individuals, indicating that female embryos were obtained at a ratio of 77%.

**[Table 3]**

| | Male | Female | Female ratio |
|---|---|---|---|
| Non-addition group | 8 | 8 | 50% |
| R848 addition group | 5 | 17 | 77% |

Sperm cultured in the same manner as in Experiment 7 were subj ected to artificial insemination, and the resulting blastocyst embryos were collected. As a result, 83.3% (5/6) of the embryos were female embryos.

### (Experiment 9) Test of Bovine Sperm

Freeze-thawed bovine sperm, thawed and washed in modified HTF medium, were cultured in modified HTF medium (Glu medium, pH 7.7, supplemented with R848 (3 nM)) (30 minutes, room temperature). Centrifugation was carried out, and then the supernatant was discarded. Subsequently, the sperm were resuspended in modified HTF medium supplemented with 1% PVP and R848 (3 nM), and then placed under PVP-free modified HTF medium to form a lower layer. Fifteen minutes later, 1.5 mL of the upper layer and 0.5 mL of the lower layer were collected. Each layer was subjected to acrosome staining, and the acrosome retention rate was determined.

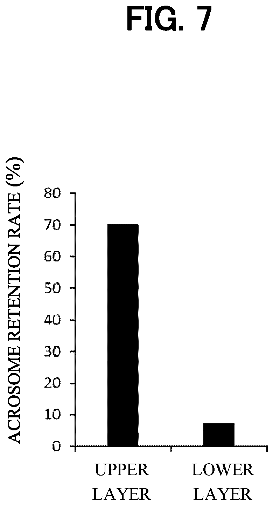

The results are illustrated in FIG. 7. The sperm in the upper layer showed an acrosome retention rate of 70%, which was significantly higher than that of the sperm in the lower layer.

The sperm collected from the lower layer were subjected to fluorescence in situ hybridization using a probe that recognizes the X chromosome, to determine the ratio between X-sperm and Y-sperm.

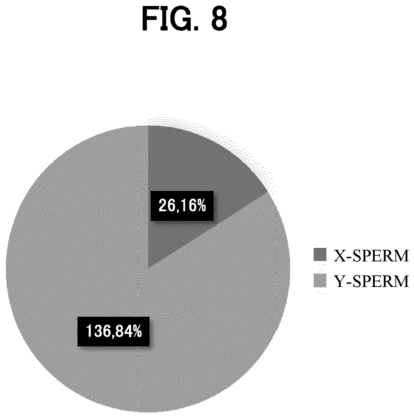

The results are illustrated in FIG. 8. Since 84% of the sperm in the lower layer were Y-sperm, it can be said that, also in the case of bovine sperm, the acrosome of Y-sperm is damaged by R848 while the acrosome of X-sperm is retained.

### (Experiment 10) Bovine In Vitro Fertilization

Freeze-thawed bovine sperm, thawed and washed in modified HTF medium, were cultured in modified HTF medium (pH 7.4, supplemented with R848 (3 nM)) (30 minutes, room temperature). Centrifugation was carried out, and then the supernatant was discarded. Subsequently, the sperm were resuspended in modified HTF medium supplemented with 1% PVP and R848 (3 nM), and then placed under PVP-free modified HTF medium to form a lower layer. Fifteen minutes later, 1.5 mL of the upper layer was collected, washed, and then subjected to in vitro fertilization. Thereafter, the resulting blastocyst embryos were subjected to sex identification by real-time PCR.

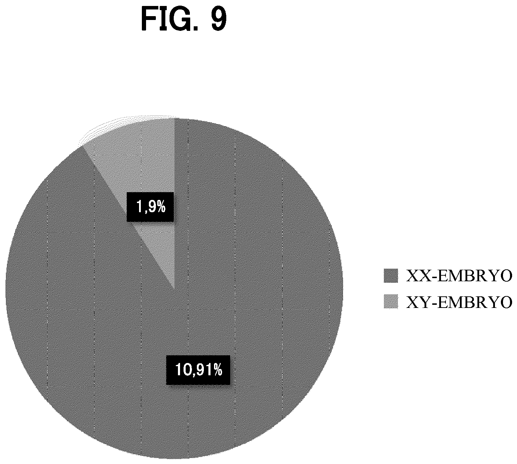

The results are illustrated in FIG. 9. The results indicate that 91% of the embryos were XX embryos, that is, female embryos.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2022-9945, filed on January 26, 2022, the entire disclosure of which is incorporated by reference herein.

### Industrial Applicability

In the method of preparing mammalian sperm according to the present disclosure, a sperm population rich in X-sperm retaining the acrosome can be obtained. Therefore, by subjecting the sperm population to artificial insemination or in vitro fertilization, male offspring can be selectively produced. The method is applicable, for example, to cows, pigs, and the like in the livestock industry.

## Claims

1. A method of preparing mammalian sperm, the method comprising culturing mammalian sperm in a medium at a pH of 7.7 to 7.9 containing a TLR7 ligand, to induce acrosome reaction of Y chromosome-bearing sperm, to obtain a sperm population rich in X-chromosome-bearing sperm retaining an acrosome.

2. The method of preparing mammalian sperm according to claim 1, wherein the medium contains albumin.

3. The method of preparing mammalian sperm according to claim 1, wherein the medium contains neither pyruvic acid nor lactic acid.

4. The method of preparing mammalian sperm according to claim 1, wherein the culture is carried out for a period longer than 30 minutes and shorter than 90 minutes.

5. The method of preparing mammalian sperm according to claim 1, wherein the TLR7 ligand is one or more selected from the group consisting of resiquimod, imiquimod, gardiquimod, and loxoribine.

6. An artificial insemination method comprising carrying out artificial insemination of a non-human mammal using non-human-mammalian sperm prepared by the method of preparing mammalian sperm according to any one of claims 1 to 5.

7. An in-vitro fertilization method comprising carrying out in vitro fertilization of a non-human mammal using non-human-mammalian sperm prepared by the method of preparing mammalian sperm according to any one of claims 1 to 5.
